# EUROPÄISCHE PATENTSCHRIFT

(11) **EP 3 409 298 B1**
(45) Veröffentlichungstag und Bekanntmachung des Hinweises auf die Patenterteilung: **07.04.2021**
(21) Anmeldenummer: 17183448.4
(22) Anmeldetag: 27.07.2017
(51) Int. Cl.: A61L 2/07

(54) **VORRICHTUNG ZUM HYGIENISIEREN UND TROCKNEN**
DEVICE FOR SANITIZING AND DRYING
DISPOSITIF D'HYGIÉNISATION ET DE SÉCHAGE

(30) Priorität: 30.05.2017 DE 102017209049
(43) Veröffentlichungstag der Anmeldung: 05.12.2018
(73) Patentinhaber: Krueger, Alexander Peter, 69118 Heidelberg (DE); Heckmann, Michael Markus, 69126 Heidelberg (DE)
(72) Erfinder: Krüger, Alexander Peter, 69118 Heidelberg (DE); Heckmann, Michael Markus, 69126 Heidelberg (DE); Schwalme, Walter, 68535 Edingen-Neckarhausen (DE)
(74) Vertreter: Patent- und Rechtsanwälte Ullrich & Naumann

(56) Entgegenhaltungen:
- EP-A1- 1 980 273
- WO-A1-93/21965
- WO-A1-2005/120593
- DE-A1-102011 114 899
- US-A- 4 544 529
- US-B1- 9 427 484

## Beschreibung

Die Erfindung betrifft eine Vorrichtung zum Hygienisieren und Trocknen mit einer Kammer, die das zu behandelnde Gut aufnimmt und mit der Außenluft zum Druckausgleich kommuniziert, mit einer im unteren Bereich der Kammer befindlichen, mit Flüssigkeit befüllbaren und zum Verdampfen der Flüssigkeit mittels eines elektrischen Heizelements beheizbaren Wanne, und mit einem elektrisch betriebenen Gebläse zur Erzeugung eines Trocknungsluftstroms durch die Kammer.

Eine solche Vorrichtung ist aus der DE 10 2011 114 899 A1 und WO 2013/050011 A1 bekannt. Die Vorrichtung ist als Haushaltsgerät zum kombinierten Desinfizieren und Trocknen von Gegenständen des alltäglichen Gebrauchs konzipiert. Exemplarisch seien Babyflaschen, Sauger, Inhalatoren und deren Teile samt Zubehör erwähnt.

Das kombinierte Desinfizieren und Trocknen von Gegenständen in ein und demselben Gerät ist auf professionellem Niveau üblich. In Kliniken, Arztpraxen, Laboratorien gibt es Autoklaven, in denen medizinische Apparate und Instrumente und Laborgeräte mit Unter- oder Überdruck, heißem Dampf, elektromagnetischer Strahlung, chemischen Desinfektions-mitteln behandelt und an Ort und Stelle getrocknet werden (vgl. die DE 698 18 617 T2). Für den Haushalt sind derartige Autoklaven zu aufwändig und auch sicherheitstechnisch nicht unbedenklich. Die Vorrichtung nach der DE 10 2011 114 899 A1 und WO 2013/050011 A1 hat deshalb erklärtermaßen kein Drucksystem, sondern eine das zu behandelnde Gut aufnehmende Kammer, die zum Druckausgleich mit der Außenluft kommuniziert.

Bei der Vorrichtung nach der DE 10 2011 114 899 A1 und WO 2013/050011 A1 ist sowohl die Wanne, die die zu verdampfende Flüssigkeit aufnimmt, als auch das Gebläse für den Trocknungsluftstrom elektrisch beheizt. Das Gebläse ist gemeinhin unter der Wanne angeordnet und der Luftausstoßstrom des Gebläses über einen zentralen Luftschacht durch die Wanne hindurch in die Kammer geleitet. Alternativ oder zusätzlich kann ein Gebläse über der Wanne angeordnet sein Das Dokument EP1982073 beschreibt eine Vorrichtung zum Desinfizieren umfassend eine Dampferzeugungseinheit mit einer elektrische Heizvorrichtung. Die Vorrichtung umfasst noch ein Gebläse, um die Trocknung zu beschleunigen und auch an schwer zugänglichen Stellen im Inneren des Liners sicherzustellen.

Aus der US 2004/0126274 A1 ist eine Vorrichtung zur Verringerung, wenn nicht Beseitigung der mikrobiologischen Population an der Oberfläche zu behandelnden Guts bekannt, bei der trockene Hitze und feuchte Hitze in einer Behandlungskammer mit ein und demselben elektrischen Heizelement erzeugt werden. Für die Erzeugung der feuchten Hitze gelangt Flüssigkeit durch die Kapillaröffnung eines Reservoirs an das Heizelement, das bei der Verdampfungstemperatur der Flüssigkeit arbeitet. Luft wird in einer Konvektionsströmung aus der Umgebung angesaugt. Wenn die Flüssigkeit restlos verbraucht ist, erfolgt bei fortgesetzter Konvektionsströmung der Luft ein automatischer Übergang auf trockene Hitze bei höherer Betriebstemperatur des Heizelements.

Aufgabe der vorliegenden Erfindung ist es, eine Vorrichtung der eingangs genannten Art in Aufbau und Handhabung zu vereinfachen.

Diese Aufgabe wird mit einer derartigen Vorrichtung dadurch gelöst, dass das Gebläse ungeheizt und der Luftausstoßstrom des Gebläses in die Wanne hinein gerichtet ist, und dass die Vorrichtung einen einzigen Einschaltzustand hat, in dem das Heizelement und das Gebläse in Betrieb sind.

Mit dem Verzicht auf eine besondere elektrische Heizung des Gebläses geht eine bauliche Vereinfachung einher. Das elektrische Heizelement für die Wanne genügt zur Erwärmung des aus dem Gebläse kommenden, in die Wanne hinein gerichteten Luftausstoßstroms. Das Trocknen mag etwas länger dauern, als bei einem elektrisch beheizten Gebläse, vollzieht sich aber jedenfalls schneller als bei bloßer Konvektionsströmung der Luft.

Vor Inbetriebnahme der Vorrichtung wird deren Wanne insbesondere per Hand, beispielsweise mittels eines Messbechers o. ä., mit destilliertem Wasser befüllt. Die Vorrichtung wird eingeschaltet zur besonders einfachen Bedienbarkeit insbesondere mittels eines einzigen Schalters, Heizelement und Gebläse gehen damit zugleich in Betrieb. Die Vorrichtung erzeugt heißen Dampf, sobald die Flüssigkeit in der Wanne die erforderliche Temperatur erreicht hat und solange Flüssigkeit in der Wanne steht.

Dadurch, dass das Gebläse in der Dampfbetriebsphase bereits läuft, wird ein Eindringen von Flüssigkeit in das Gebläse verhindert und die Dampf/Luftströmung verstärkt.

Wenn die Flüssigkeit in der Wanne restlos verdampft ist, erfolgt ein automatischer Übergang in die Trocknungsphase, in der Heizelement und Gebläse einfach weiterlaufen. Die Abschaltung erfolgt von Hand oder mittels einer Zeitschaltuhr. Die Vorrichtung kann eine Zustandsanzeige mittels LED aufweisen.

Heizung und Gebläse der Vorrichtung werden zugleich ein- und ausgeschaltet. Ein einziger Bedienschalter genügt. Heizung und Gebläse können über die Dampfbetriebs- und Trocknungsphase bei voller elektrischer Leistungsaufnahme einfach durchlaufen. Die Steuerung der Vorrichtung gestaltet sich dadurch sehr einfach.

Folglich ist mit der beanspruchten Vorrichtung zum Hygienisieren und Trocknen eine Vorrichtung realisiert, deren Aufbau und Handhabung vereinfacht sind.

Bei einer bevorzugten Ausführungsform ist das Gebläse an einen Luftausstoßkanal angeschlossen, der im Randbereich der Wanne einen in die Wanne hinein gerichteten Mündungsstutzen mit einer Luftaustrittsöffnung oberhalb der Wanne hat.

Bei einer bevorzugten Ausführungsform ist das Gebläse seitlich oberhalb der Wanne angeordnet und der Mündungsstutzen des Luftausstoßkanals abwärts gekrümmt.

Bei einer bevorzugten Ausführungsform ist die Wanne zylinderbecher-förmig. Die Wanne hat einen umlaufenden, radial nach außen abstehenden Randflansch, mit dem sie auf einer Unterlage aus Kunststoff ruht. Der Mündungsstutzen des Luftausstoßkanals ist einstückig an die Unterlage angeformt.

Bei einer bevorzugten Ausführungsform weist die Vorrichtung oberhalb der Wanne einen Korb für das zu behandelnde Gut auf. Der Korb passt mit einer Boden- und Wandeinbuchtung über den Mündungsstutzen des Luftausstoßkanals und ist dadurch zentriert.

Bei einer bevorzugten Ausführungsform hat die Vorrichtung eine den Korb überdeckende Haube, die mit einem Wandausschnitt über den Mündungsstutzen des Luftausstoßkanals passt und dadurch zentriert ist.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: die perspektivische Ansicht einer Vorrichtung zum Hygienisieren und Trocknen;
- Fig. 2: eine Draufsicht von oben auf die Vorrichtung;
- Fig. 3: eine Vorderansicht der Vorrichtung;
- Fig. 4: einen Längsschnitt durch die Vorrichtung nach A - A von Fig. 3; und
- Fig. 5: eine perspektivische Explosionszeichnung der Vorrichtung.

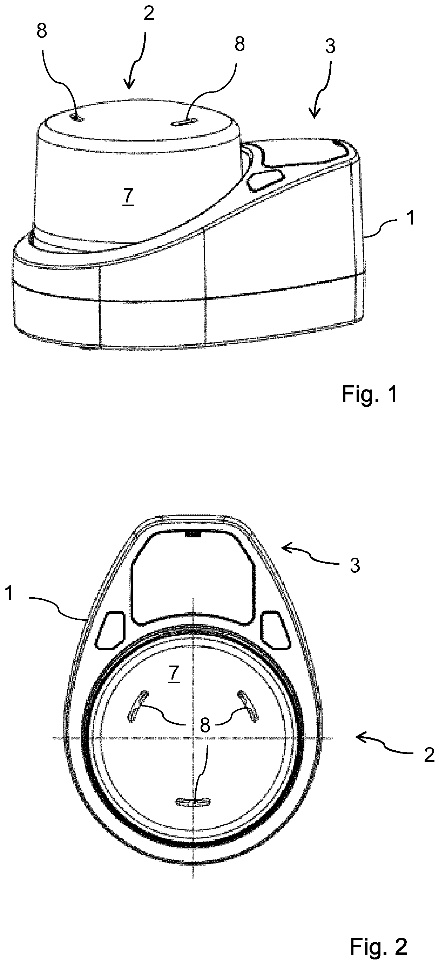

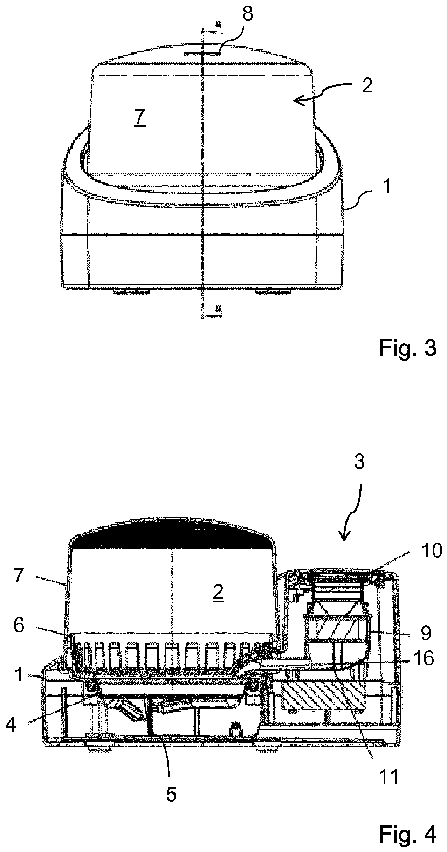

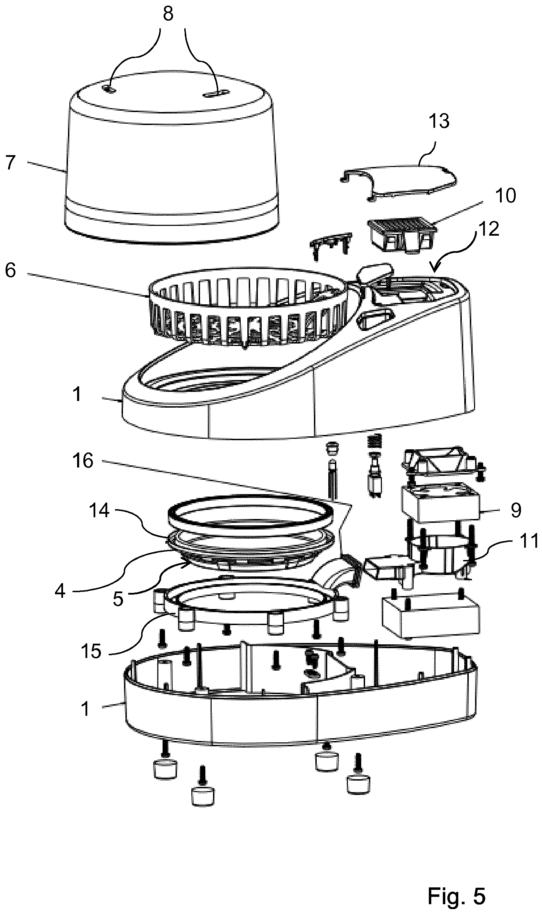

Die Figuren 1 bis 5 zeigen ein bevorzugtes Ausführungsbeispiel einer erfindungsgemäßen Vorrichtung zum Hygienisieren und Trocknen. Die Vorrichtung hat ein im Grundriss tropfenförmiges Gehäuse 1, das an der Breitseite einen hohen zylindrischen Behandlungsraum 2 für das zu hygienisierende und zu trocknende Gut und an der Schmalseite eine niedrigere Luftansaugpartie 3 bildet. Bei dem Gehäuse 1 handelt es sich um ein zweiteiliges Kunststoffgehäuse.

Im Konkreten weist die Vorrichtung eine Kammer 2 auf, die das zu behandelnde Gut aufnimmt und mit der Außenluft zum Druckausgleich kommuniziert. Des Weiteren befindet sich im unteren, d.h. im Bodenbereich des Behandlungsraums bzw. der Kammer 2 eine Wanne 4, die elektrisch beheizt ist, um Wasser daraus zu verdampfen. Bei dem elektrischen Heizelement handelt es sich um eine Heizplatte 5, die unterhalb der Wanne 4 angeordnet ist.

Über der Wanne 4 sitzt ein Korb 6, der das zu behandelnde Gut aufnimmt. Der Behandlungsraum bzw. die Kammer 2 ist nach außen durch eine aufgestülpte Haube 7 abgedeckt. Die Haube 7 hat oben Schlitze 8 für den zum Druckausgleich stattfindenden Durchtritt von Luft und Dampf.

Kernstück der Luftansaugpartie 3 ist ein Gebläse 9, mit dem Luft von oben durch einen Filter 10 angesaugt und nach unten in einen Umlenktopf 11 mit einem radialen Abgang ausgestoßen wird. Der Filter sitzt auswechselbar in einer Filterkammer 12, die nach oben durch ein Gitter 13 abgedeckt ist.

Die Wanne 4 hat die Form eines flachen Zylinderbechers mit einem radialen nach außen abstehenden Randflansch 14. Mit diesem Randflansch 14 ruht die Wanne 4 auf einem Haltering 15 aus Kunststoff. An den Haltering 15 ist einstückig ein Mündungsstutzen 16 zur Luftführung für den Luftausstoßkanal angeformt. Der Mündungsstutzen 16 befindet sich am Rand der Wanne 2. Er ist abwärts gekrümmt und in die Wanne 4 hinein gerichtet.

Der Mündungsstutzen 16 hat eine Luftaustrittsöffnung oberhalb des Flüssigkeitspegels in der Wanne 4. In das andere Ende des Mündungsstutzens 16 wird der Abgang des Umlenktopfes 11 für den Luftstrom eingesteckt.

Die Wanne 4 ist von unten elektrisch beheizt. Das Gebläse 9 zur Erzeugung eines Trocknungsluftstroms durch die Kammer 2 ist ungeheizt, wobei der Luftausstoßstrom des Gebläses 9 in die Wanne 4 hineingerichtet ist. Der Luftstrom erfährt eine Erwärmung allein durch die Heizung der Wanne 4.

Der Korb 6 hat eine Boden- und Wandeinbuchtung, mit der er über den Mündungsstutzen 16 des Luftausstoßkanals passt. Durch diesen Passsitz ist der Korb 6 zentriert.

Die Haube 7 hat einen Wandausschnitt, mit dem sie über den Mündungsstutzen 16 des Luftausstoßkanals passt. Durch diesen Passsitz ist die Haube 7 zentriert.

Die Vorrichtung hat einen einzigen Einschaltzustand, in dem sowohl das Heizelement 5 als auch das Gebläse 9 in Betrieb sind. Hierzu ist zur einfachen Bedienbarkeit lediglich ein einziger, in der Fig. nicht gezeigter Schalter erforderlich. Die Zustandsanzeige erfolgt über eine in der Fig. nicht gezeigte LED-Anzeige.

Das bevorzugte Ausführungsbeispiel einer Vorrichtung zum Hygienisieren und Trocknen eignet sich ideal für den Privatgebrauch, d.h. als Haushaltsgerät zum kombinierten Desinfizieren und Trocknen von Gegenständen des alltäglichen Gebrauchs.

Zur Vermeidung von Wiederholungen sei in Bezug auf Merkmale, die sich den Figuren nicht entnehmen lassen, auf den allgemeinen Teil der Beschreibung verwiesen.

Schließlich sei angemerkt, dass das voranstehend beschriebene Ausführungsbeispiel lediglich zur beispielhaften Erörterung der beanspruchten Lehre dient, diese jedoch nicht auf das Ausführungsbeispiel einschränkt.

### Bezugszeichenliste

- 1: Gehäuse
- 2: Behandlungsraum / Kammer
- 3: Luftansaugpartie
- 4: Wanne
- 5: Heizplatte / Heizelement
- 6: Korb
- 7: Haube
- 8: Schlitze
- 9: Gebläse / Lüfter
- 10: Filter
- 11: Umlenktopfes
- 12: Filterkammer
- 13: Gitter
- 14: Randflansch
- 15: Haltering / Unterlage aus Kunststoff
- 16: Mündungsstutzen für den Lustausstoßkanal

## Patentansprüche

1. Vorrichtung zum Hygienisieren und Trocknen mit einer Kammer (2), die das zu behandelnde Gut aufnimmt und mit der Außenluft zum Druckausgleich kommuniziert, mit einer im unteren Bereich der Kammer (2) befindlichen, mit Flüssigkeit befüllbaren und zum Verdampfen der Flüssigkeit mittels eines elektrischen Heizelements (5) beheizbaren Wanne (4), und mit einem elektrisch betriebenen Gebläse (9) zur Erzeugung eines Trocknungsluftstroms durch die Kammer (2),
**dadurch gekennzeichnet, dass** nur die Wanne (4) ein elektrisches Heizelement (5) aufweist und der Luftausstoßstrom des Gebläses (9) in die Wanne (4) hinein gerichtet ist und der Luftaustoßstrom eine Erwärmung alleine durch die Heizung der Wanne (4) erfährt, wobei die Vorrichtung derart ausgeführt ist, dass das Heizelement (5) und das Gebläse (9) ausschließlich zugleich ein- und ausschaltbar sind., und wobei das Gebläse (9) an einen Luftausstoßkanal angeschlossen ist, der im Randbereich der Wanne (4) einen in die Wanne (4) hinein gerichteten Mündungsstutzen (16) mit einer Luftaustrittsöffnung oberhalb der Wanne (4) hat.

2. Vorrichtung nach Anspruch 1, **dadurch gekennzeichnet, dass** das Gebläse (9) seitlich oberhalb der Wanne (4) angeordnet und der Mündungsstutzen (16) des Luftausstoßkanals abwärts gekrümmt ist.

3. Vorrichtung nach Anspruch 2, **dadurch gekennzeichnet, dass** die Wanne (4) zylinderbecherförmig ist und einen umlaufenden, radial nach außen abstehenden Randflansch (14) hat, mit dem die Wanne (4) auf einer Unterlage (15) aus Kunststoff ruht, und dass der Mündungsstutzen (16) des Luftausstoßkanals einstückig an die Unterlage (15) angeformt ist.

4. Vorrichtung nach einem der Ansprüche 2 oder 3, **dadurch gekennzeichnet, dass** sie oberhalb der Wanne (4) einen Korb (6) für das zu behandelnde Gut aufweist, und dass der Korb (6) mit einer Boden- und Wandeinbuchtung über den Mündungsstutzen (16) des Luftausstoßkanals passt.

5. Vorrichtung nach Anspruch 4, **dadurch gekennzeichnet, dass** sie eine den Korb (6) überdeckende Haube (7) hat, und dass die Haube (7) mit einem Wandausschnitt über den Mündungsstutzen (16) des Luftausstoßkanals passt.

## Claims

1. Device for sanitising and drying having a chamber (2) which receives the product which is intended to be processed and which communicates with the ambient air for pressure compensation, having a tank (4) which is located in the lower region of the chamber (2) and which can be filled with liquid and which can be heated by means of an electric heating element (5) in order to evaporate the liquid, and having an electrically operated fan (9) in order to produce a drying air flow through the chamber (2),
**characterised in that** only the tank (4) has an electric heating element (5) and the air discharge flow of the fan (9) is directed into the tank (4) and the air discharge flow is subjected to heating only as a result of the heating of the tank (4), wherein the device is configured in such a manner that the heating element (5) and the fan (9) can be switched on and off exclusively at the same time, and wherein the fan (9) is connected to an air discharge channel which has in the edge region of the tank (4) an opening nozzle (16) which is directed into the tank (4) and which has an air discharge opening above the tank (4).

2. Device according to claim 1, **characterised in that** the fan (9) is arranged laterally above the tank (4) and the opening nozzle (16) of the air discharge channel is curved downwards.

3. Device according to claim 2, **characterised in that** the tank (4) is in the form of a cylindrical cup and has a peripheral, radially outwardly protruding edge flange (14) with which the tank (4) rests on a base (15) of plastics material, and **in that** the opening nozzle (16) of the air discharge channel is formed integrally on the base (15).

4. Device according to either claim 2 or 3, **characterised in that** it has above the tank (4) a basket (6) for the product which is intended to be processed and **in that** the basket (6) fits with a base and wall recess over the opening nozzle (16) of the air discharge channel.

5. Device according to claim 4, **characterised in that** it has a cover (7) which covers the basket (6), and **in that** the cover (7) fits with a wall cut-out over the opening nozzle (16) of the air discharge channel.

## Revendications

1. Dispositif de désinfection et de séchage avec une chambre (2), qui contient le produit à traiter et qui communique avec l'air extérieur pour la compensation de la pression, avec une cuve (4) se trouvant dans la partie inférieure de la chambre (2), pouvant être remplie de liquide et pouvant être chauffée, pour l'évaporation du liquide, au moyen d'un élément chauffant électrique (5), et avec un ventilateur électrique (9) pour la production d'un flux d'air de séchage à travers la chambre (2),
**caractérisé en ce que** seule la cube (4) comprend un élément chauffant électrique (5) et le flux d'air sortant du ventilateur (9) est orienté vers l'intérieur de la cuve (4) et le flux d'air sortant subit un chauffage seul du fait du chauffage de la cuve (4), dans lequel le dispositif est conçu de façon à ce que l'élément chauffant (5) et le ventilateur (9) puissent être activés et désactivés exclusivement en même temps et dans lequel le ventilateur (9) est raccordé à un canal d'éjection d'air qui présente, sur le bord de la cuve (4), une tubulure d'embouchure (16) orientée vers l'intérieur de la cuve (4), avec une ouverture de sortie d'air au-dessus de la cuve (4).

2. Dispositif selon la revendication 1, **caractérisé en ce que** le ventilateur (9) est disposé latéralement au-dessus de la cuve (4) et la tubulure d'embouchure (16) du canal de sortie d'air est incurvée vers le bas.

3. Dispositif selon la revendication 2, **caractérisé en ce que** la cuve (4) présente la forme d'un gobelet cylindrique et comprend une bride de bord (14) circulaire dépassant radialement vers l'extérieur, avec laquelle la cuve (4) repose sur un support (15) constitué de matière plastique et **en ce que** la tubulure d'embouchure (16) du canal d'éjection d'air est moulé d'une seule pièce sur le support (15).

4. Dispositif selon l'une des revendications 2 ou 3, **caractérisé en ce qu'**il comprend, au-dessus de la cuve (4), un panier (6) pour le produit à traiter et **en ce que** le panier (6) s'adapte, avec une échancrure dans le fond et dans la paroi, au-dessus de la tubulure d'embouchure (16) du canal d'éjection d'air.

5. Dispositif selon la revendication 4, **caractérisé en ce qu'**il comprend un capot (7) recouvrant le panier (6) et **en ce que** le capot (7) s'adapte, avec une découpe dans la paroi, au-dessus de la tubulure d'embouchure (16) du canal d'éjection d'air.
